Europäisches Patentamt

European Patent Office

Office européen des brevets

(19)

(11) Veröffentlichungsnummer: **0 172 262**
**B1**

(12)                    # EUROPÄISCHE PATENTSCHRIFT

(45) Veröffentlichungstag der Patentschrift:
25.01.89

(21) Anmeldenummer: 84109224.0

(22) Anmeldetag: 03.08.84

(51) Int. Cl.⁴: **A 61 F 2/36**

(54) **Femurkopfprothese.**

(30) Priorität: 15.11.83 CH 6127/83

(43) Veröffentlichungstag der Anmeldung:
26.02.86 Patentblatt 86/9

(45) Bekanntmachung des Hinweises auf die Patenterteilung:
25.01.89 Patentblatt 89/4

(84) Benannte Vertragsstaaten:
AT DE FR IT

(56) Entgegenhaltungen:
DE-A-2 524 923
US-A-3 102 536

(73) Patentinhaber: GEBRÜDER SULZER
AKTIENGESELLSCHAFT, Zürcherstrasse 9, CH-
8401 Winterthur (CH)

(72) Erfinder: Frey, Otto, Wallrütistrasse 56, CH- 8404
Winterthur (CH)

(74) Vertreter: Dipl.- Ing. H. Marsch Dipl.- Ing. K.
Sparing Dipl.- Phys.Dr. W.H. Röhl
Patentanwälte, Rethelstrasse 123, D-4000
Düsseldorf 1 (DE)

EP 0 172 262 B1

## Beschreibung

Die Erfindung betrifft eine Femurkopfprothese, zusammengesetzt aus einem Gelenkkopf und einem Verankerungsschaft, wobei der Gelenkkopf einen sich in ihn hinein verjüngenden Mutterkonus zur Aufnahme eines einen Vaterkonus bildenden Zapfen des Verankerungsschaftes aufweist.

Eine vielfach verwendete Massnahme, um den Gelenkkopf einer Kugelgelenk-Endoprothese, insbesondere eine Femurkopfprothese, mit dem Verankerungsschaft zu verbinden, besteht in einer Konussteckverbindung, bei der der Gelenkkopf über einen, gegbenenfalls selbsthemmenden, Konus auf einen Zapfen eines Verankerungsschaftes aufgesteckt ist (DE-OS 25 48 077). Besonders bei relativ grossen Gelenkköpfen mit erhöhten Torsionsmomenten, wie sie für sogenannte Frakturprothesen verwendet werden, kommt es nach der Implantation häufig zu unerwünschten Relativdrehungen zwischen Gelenkkopf und Zapfen.

Weiterhin ist bekannt (CH-PS-507 704; DE-PS-2 220 304), über eine ähnliche konische Steckverbindung einen Gelenkkopf auf einem Zapfen zu befestigen, bei der die Zapfenachse nicht mit der Symmetrieachse des Gelenkkopfes zusammenfällt, um die Stellung des Gelenkkopfes relativ zur Femurachse an die individuellen Unterschiede im Skelettbau der verschiedenen Patienten annähern zu können. Bei derartigen Prothesen mit "schiefer" Stellung der beiden Achsen muss gewährleistet sein, dass sich der Gelenkkopf nicht relativ zum Zapfen während der "Lebenszeit" der Protehse verdrehen kann. Daher ist es bekannt, am Fuss des Zapfens als Rippen, Nasen, Zähne oder ähnliches ausgebildete Verdrehsicherungen vorzusehen, die in entsprechende radiale Nuten im Mantel des Mutterkonus eingreifen. Ganz abgesehen, dass die Herstellung der Nuten im Mutterkonus einen hohen zusätzlichen Aufwand erfordert, hat diese Verdrehsicherung den Nachteil, dass sie keine stufenlose Einstellung des Gelenkkopfes relativ zum Zapfen erlaubt, sondern nur eine der Anzahl der Nuten entsprechende relative Drehung und Fixierung des Kugelkopfes gegenüber dem Zapfen ermöglicht.

Aufgabe der Erfindung ist es, eine Verdrehsicherung zu schaffen, mit der ein schalenförmiger Gelenkkopf vom Operateur ohne Beachtung einer bestimmten Winkelstellung zwischen Zapfen und Gelenkkopf auf dem Zapfen befestigt werden kann, und die darüberhinaus stufenlos einstellbar gegenüber dem Zapfen des Verankerungsschaftes ist. Mit der vorliegenden Erfindung wird diese Aufgabe dadurch gelöst, dass der Gelenkkopf schalenförmig auf eine Hülse aufgesetzt und mit ihr verschweisst ist, und dass ferner im unzusammengesetzten Zustand der Mutterkonus am verjüngten Ende der Hülse einen Bereich aufweist, dessen Konuswinkel $\beta$ grösser als der Konuswinkel $\alpha$ seines schaftnahen Bereichs ist, in dem der Winkel des Mutterkonus gleich dem über die ganze Zapfenhöhe konstanten Winkel $\alpha$ des Vaterkonus ist, und dass schliesslich der Zapfen im zusammengesetzten Zustand in den Bereich der Hülse mit dem grösseren Konuswinkel hineinreicht.

Beim Aufsetzen und Einschlagen oder -pressen des Zapfens in die Hülse weitet sich deren verjüngtes Ende auf und bewirkt eine Klemmverbindung mit relativ grosser Haftfläche zwischen beiden Konen.

Das Aufweiten der Hülse kann erleichtert werden, wenn über die Tiefe des Bereichs mit dem grösseren Konuswinkel Radialnuten auf dem Umfang der Hülse verteilt sind; um jede Möglichkeit eines Eindringens von Körperflüssigkeit in den schalenförmigen Gelenkkopf - entlang der Konussteckverbindung - auszuschliessen, ist es vorteilhaft, wenn das verjüngte Ende der Hülse durch einen Deckel verschlossen ist, der die Radialnuten haubenartig abdeckt.

Die neue Hüftgelenkprothese besteht in erster Linie aus Metall, wobei alle für Endoprothesen üblichen Metalle und oder Legierungen verwendet werden können.

Im folgenden wird die Erfindung anhand eines Ausführungsbeispiels im Zusammenhang mit der Zeichnung näher erläutert.

Fig. 1　zeigt in einem Schnitt I-I von Fig. 2 die den Mutterkonus enthaltende Hülse, auf die der Gelenkkopf aufgesetzt wird;

Fig. 2　ist eine Aufsicht auf Fig. 1 von oben;

Fig. 3　gibt in einem Schnitt III-III von Fig. 4 den auf den Zapfen eines Verankerungsschaftes aufgepressten Gelenkkopf wieder, während

Fig. 4　der Schnitt IV-IV von Fig. 3 ist.

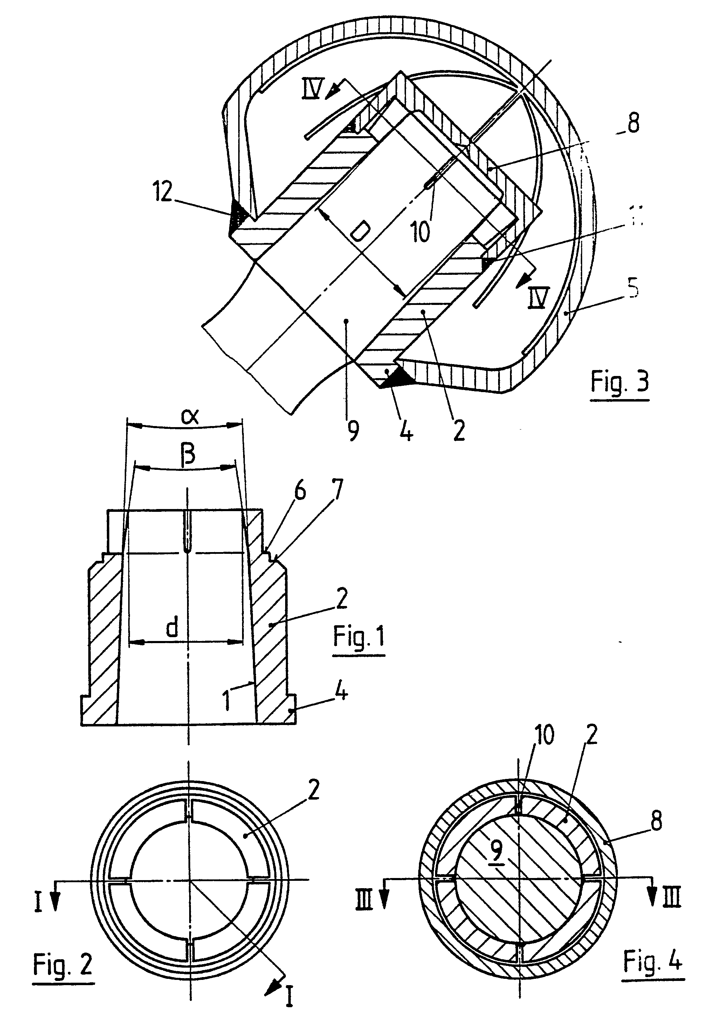

Der Mutterkonus 1 (Fig. 1) befindet sich in einer Hülse 2, die am unteren Ende einen Absatz 4 für die Abstützung eines mit ihr verschweissten Gelenkkopfes 5 (Fig. 3) hat. Das obere Ende der Hülse 2 besitzt aussen ebenfalls stufenförmige Absätze 6 und 7, auf denen ein Deckel 8 (Fig. 3) gelagert ist.

Gemäss der vorliegenden Erfindung ist der Konuswinkel $\beta$ des Mutterkonus 1 am verjüngten oberen Ende der Hülse 2 gegenüber demjenigen $\alpha$ im schaftnahen Bereich der Hülse 2 vergrössert. Der Konuswinkel $\alpha$ im schaftnahen Bereich des Mutterkonus 1 stimmt dabei mit dem Winkel des Vaterkonus des Zapfens 9 (Fig. 3) überein. Beispielsweise beträgt der Durchmesser d des Mutterkonus 1 am verjüngten Ende 14 mm, während derjenige D (Fig. 3) des Zapfens 9 - bei einer Toleranz von 1 - 2/100 mm - 14,2 mm ist.

Um das Aufweiten beim Einpressen des Zapfens 9 - wobei sich das verjüngte Ende der Hülse 2 plastisch verformt - zu erleichtern, sind im Bereich der Hülse 2, dessen Konuswinkel $\beta$ vergrössert ist, radiale Nuten 10 eingefräst.

Wie ein Vergleich der Fig. 2 und 4 erkennen lässt, sind die Nuten 10 bei der noch nicht auf dem Zapfen 9 aufgesteckten Hülse 2 breiter als bei der fertig montierten Prothese (Fig. 4).

Das Gleiche gilt für den Radialspalt zwischen dem oberen Ende der Hülse 2 und dem Deckel 8.

Bei der Fabrikation wird das verjüngte Ende der Hülse 2 nach dem Einschneiden der Nuten 10 zunächst mit dem Deckel 8 mit Hilfe der Schweissnaht 11 (Fig. 3) flüssigkeltsdicht verschlossen, ehe der Gelenkkopf 5 aufgesetzt und ebenfalls durch eine Schweissnaht 12 (Fig. 3) mit der Hülse 2 verbunden wird.

## Patentansprüche

1. Femurkopfprothese, zusammengesetzt aus einem Gelenkkopf und einem Verankerungsschaft, wobei der Gelenkkopf einen sich in ihn hinein verjüngenden Mutterkonus zur Aufnahme eines einen Vaterkonus bildenden Zapfen des Verankerungsschaftes aufweist, dadurch gekennzeichnet, dass der Gelenkkopf (5) schalenförmig, auf eine Hülse (2) aufgesetzt und mit ihr verschweisst ist, und dass ferner im unzusammengesetzten Zustand der Mutterkonus (1) am verjüngten Ende der Hülse (2) einen Bereich aufweist, dessen Konuswinkel ($\beta$) grösser als der Konuswinkel ($\alpha$) seines schaftnahen Bereichs ist, in dem der Winkel des Mutterkonus gleich dem über die ganze Zapfenhöhe konstanten Winkel ($\alpha$) des Vaterkonus ist, und dass schliesslich der Zapfen (9) im zusammengesetzten Zustand in den Bereich der Hülse (2) mit dem grösseren Konuswinkel ($\beta$) hineinreicht.

2. Femurkopfprothese nach Anspruch 1, dadurch gekennzeichnet, dass über die Tiefe des Bereichs mit dem grösseren Konuswinkel ($\beta$) Radialnuten (10) auf dem Umfang der Hülse (2) verteilt sind.

3. Femurkopfprothese nach Anspruch 1, dadurch gekennzeichnet, dass das verjüngte Ende der Hülse (2) durch einen Deckel (8) verschlossen ist, der die Radialnuten (10) haubenartig abdeckt.

## Claims

1. A femur head prosthesis combined from a joint head and an anchoring shank, the head having a female cone which narrows into the head and which is adapted to receive a pin of the anchoring shank, the pin forming a male cone, characterised in that the head (5) is placed shell-fashion on a sleeve (2) and welded thereto and in the unassembled state the female cone (1) has at the narrowed end of the sleeve (2) a zone whose cone angle ($\beta$) is greater than the cone angle ($\alpha$) of its zone near the shank, in which zone the angle of the female cone is equal to the male cone angle ($\alpha$), the same being constant over the whole height of the pin, and the pin (9) in the assembled state extends into the zone of the sleeve (2) having the greater cone angle ($\beta$).

2. A prosthesis according to claim 1, characterised in that radial grooves (10) are distributed around the periphery of the sleeve (2) over the depth of the zone having the larger cone angle ($\beta$).

3. A prosthesis according to claim 1, characterised in that the narrowed end of the sleeve (2) is sealed by a cover (8) which covers the radial grooves (10) like a hood.

## Revendications

1. Prothèse pour tête de fémur, se composant d'une tête d'articulation et d'une tige d'ancrage, la tête d'articulation présentant un cône femelle se rétrécissant vers l'intérieur de cette tête afin de recevoir un tenon de la tige d'ancrage, qui forme un cône mâle, caractérisée par le fait que la tête d'articulation (5) est placée, en forme de calotte, sur une douille (2) à laquelle elle est soudée; par le fait que, à l'état non assemblé, le cône femelle (1) présente en outre, à l'extrémité rétrécie de la douille (2), une région dont l'angle de conicité ($\beta$) est plus grand que l'angle de conicité ($\alpha$) de sa région proche de la tige, dans laquelle l'angle du cône femelle est égal à l'angle ($\alpha$) du cône mâle, constant sur toute la hauteur du tenon; et, enfin, par le fait que le tenon (9) pénètre, à l'état assemblé, dans la région de la douille (2) présentant l'angle de conicité supérieur ($\beta$).

2. Prothèse pour tête de fémur selon la revendication 1, caractérisée par le fait que des rainures radiales (10) sont réparties sur le pourtour de la douille (2), sur la profondeur de la région présentant l'angle de conicité supérieur ($\beta$).

3. Prothèse pour tête de fémur selon la revendication 1, caractérisée par le fait que l'extrémité rétrécie de la douille (2) est obturée par l'intermédiaire d'un couvercle (8), qui recouvre les rainures radiales (10) à la manière d'un capot.

Fig. 3

Fig. 1

Fig. 2

Fig. 4